Europäisches Patentamt
European Patent Office
Office européen des brevets

(19)

(11) Veröffentlichungsnummer: 0 243 841
A1

(12) EUROPÄISCHE PATENTANMELDUNG

(21) Anmeldenummer: 87105793.1

(51) Int. Cl.[4]: C07D 221/14 , A61K 31/435

(22) Anmeldetag: 18.04.87

(30) Priorität: 29.04.86 DE 3614414

(43) Veröffentlichungstag der Anmeldung:
04.11.87 Patentblatt 87/45

(84) Benannte Vertragsstaaten:
AT BE CH DE ES FR GB IT LI NL SE

(71) Anmelder: KNOLL AG
Knollstrasse
D-6700 Ludwigshafen(DE)

(72) Erfinder: Brana, Miguel Fernandez, Dr.
Avenida de Burgos No.20, 1.M.
E-28036 Madrid(ES)
Erfinder: Berlanga, José Maria Castellano, Dr.
Virgen del Sagrario 18
Madrid(ES)
Erfinder: Schlick, Erich, Dr.
Rudolph-Wihr-Strasse 4 1/2
D-6708 Neuhofen(DE)
Erfinder: Keilhauer, Gerhard, Dr.
Industriestrasse 20
D-6701 Dannstadt-Schauernheim(DE)

(74) Vertreter: Karau, Wolfgang Dr. et al
BASF Aktiengesellschaft Carl-Bosch-Strasse
38
D-6700 Ludwigshafen(DE)

(54) Neue Benzo de isochinolin-1,3-dione, ihre Herstellung und Verwendung.

(57) Es werden Verbindungen der Formel

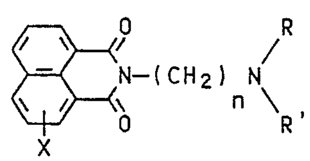

I,

worin X, n, R und R' die in der Beschreibung angegebene Bedeutung besitzen, und deren Herstellung beschrieben. Die Verbindungen eignen sich zur Bekämpfung von Krankheiten.

EP 0 243 841 A1

## Neue Benzo[de]isochinolin-1,3-dione, ihre Herstellung und Verwendung

Die vorliegende Erfindung betrifft neue Benzo[de]isochinolin-1,3-dione, Verfahren zu ihrer Herstellung und deren Verwendung in Arzneimitteln mit tumorhemmenden Eigenschaften.

Es ist bereits bekannt, daß bestimmte Benzo[de]isochinolin-1,3-dione eine Antitumorwirkung besitzen (Afinidad XXXV, 105 (1978), Cancer Chemother. and Pharmacol. 4, 61 (1980), Eur. J. Med. Chem. - Chim. Ther. 16, 207 (1981), J. Med. Chem. 27, 450 (1984), dto. 28, 1216 (1985), US-PS 4 146 720, US-PS 4 204 063, US-PS 4 499 266).

Es wurde nun gefunden, daß Benzo[de]isochinolin-1,3-dione der Formel I

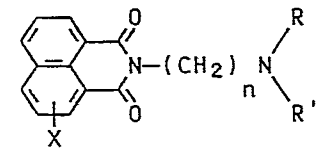

I,

worin

X eine Hydroxy-, Nitro-, $C_{1-4}$-Alkoxy-, Amino-, $C_{1-4}$-Alkylamino, Di-$C_{1-4}$-Alkylamino-, $C_{1-4}$-Alkylcarbonylamino-, $C_{1-4}$-Alkoxycarbonylamino-, $C_{1-6}$-Alkyl-oder Trifluormethylgruppe oder ein Wasserstoff-oder Halogenatom darstellt,

n die Zahl 0, 1, 2, 3 oder 4 bedeutet

R ein Wasserstoffatom oder eine Hydroxy-$C_{1-4}$-alkylgruppe ist und

R′ eine Hydroxy-$C_{1-4}$-alkylgruppe darstellt,

wobei jedoch nicht X eine 5-Nitrogruppe oder ein Wasserstoffatom und n nicht 2 sein kann, wenn R ein Wasserstoffatom und R′ eine Hydroxyethylgruppe darstellt, sowie deren Salze mit physiologisch verträglichen Säuren eine günstigere Wirkung besitzen.

Eine besonders günstige Wirkung besitzen die Verbindungen der Formel I, in der X eine Nitro-, Alkoxycarbonylamino-, Alkylamino-oder Dialkylaminogruppe oder ein Chloratom, n die Zahlen 2 oder 3, R ein Wasserstoffatom oder eine Hydroxyethylgruppe und R′ eine Hydroxyethylgruppe darstellen. Der Substituent X steht bevorzugt in der 5-oder 6-Stellung.

Als physiologisch verträgliche Säuren eignen sich zur Salzbildung mit den neuen Verbindungen insbesondere Salzsäure, Schwefelsäure, Phosphorsäure, Essigsäure, Malonsäure, Bernsteinsäure, Fumarsäure, Maleinsäure, Zitronensäure, Weinsäure, Milchsäure, Amidosulfonsäure und Oxalsäure.

Die neuen Verbindungen lassen sich herstellen, indem man Naphthalsäureanhydride der Formel II

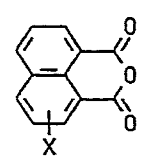

II,

worin X die angegebene Bedeutung besitzt, mit einem Amin der Formel III

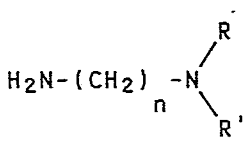

III,

worin n, R und R′ die angegebene Bedeutung besitzen, umsetzt und die so erhaltenen Verbindungen gegebenenfalls in ihre Salze mit physiologisch verträglichen Säuren überführt.

Die Umsetzung der Verbindungen II und III erfolgt vorzugsweise in einem Lösungsmittel bei Temperaturen zwischen 0°C und der Siedetemperatur des Lösungsmittels, vorzugsweise jedoch bei Raumtemperatur. Als Lösungsmittel eignen sich beispielsweise niedere Alkohole. Das gewünschte Produkt I fällt aus dem Lösungsmittel aus und kann durch Umkristallisieren oder chromatographisch weiter gereinigt werden.

Die neuen Verbindungen besitzen eine gute cytotoxische Wirkung wie sich in folgendem Versuch zeigen läßt: Tumorzellen menschlicher Herkunft werden auf Mikrotiterplatten in einer Konzentration von 1 bis 5 x $10^3$ Zellen pro Vertiefung ausplattiert und im Brutschrank bei 37°C und unter Normalatmosphäre, jedoch unter Zusatz von 5 % Kohlendioxid, über Nacht in einem Vollmedium vorinkubiert. Die zu testenden Substanzen werden in demselben Vollmedium gelöst und aus den Lösungen Verdünnungsreihen (Verdünnungsverhältnis 1:2) hergestellt. Jeweils 0,1 ml der so erhaltenen Lösungen werden in die Vertiefungen der Mikrotiterplatten gegeben. Die Platten werden erneut für 72 h bei 37°C inkubiert. Danach werden adhärente Zellen mit Kristallviolett und nicht-adhärente Zellen mit MTT (Tetrazolium) angefärbt und deren Extinktion bei 540 nm gemessen. Die Abnahme der Extinktion gegenüber einem Blindwert (erhalten ohne Testsubstanz) ist ein Maß für Cytotoxizität der Verbindungen.

Die neuen Verbindungen eignen sich daher zur Behandlung von festen Tumoren wie auch von bestimmten Formen von Leukämie.

Die folgenden Beispiele erläutern die Erfindung näher.

Beispiel 1

Eine Mischung aus 4,8 g (0,02 mol) 3-Nitro-1,8-naphthalsäure und 3,3 g (0,02 mol) N-(3-Aminopropyl)-diethanolamin wird in 50 ml wasserfreiem Ethanol bei Raumtemperatur 5 h gerührt. Der Niederschlag wird abfiltriert und aus Ethanol umkristallisiert. Man erhält in einer Ausbeute von 83 % 2-[3-(Di(2-hydroxyethyl)-amino)-propyl]-5-nitrobenzo[de]isochinolin-1,3-dion, Fp. 124-126°C.

Analog Beispiel 1 wurde hergestellt:

2. 2-[3-[Di(2-hydroxyethyl)amino]propyl]-6-nitrobenzo[de]isochinolin-1,3-dion, Fp. 88°C (Ethanol),

3. 2-[3-[Di(2-hydroxyethyl)amino]propyl]benzo[de]isochinolin-1,3-dion, Fp. 101-103°C (Essigsäureethylester),

4. 5-Amino-2-[3-[di(2-hydroxyethyl)amino]propyl]benzo[de]isochinolin-1,3-dion, Fp. 142°C (Ethanol),

5. 6-Amino-2-[3-[di(2-hydroxyethyl)amino]propyl]benzo[de]isochinolin-1,3-dion, Fp. 158-160°C (Methanol),

6. 6-Chlor-2-[3-[di(2-hydroxyethyl)amino]propyl]benzo[de]isochinolin-1,3-dion, Fp. 118-120°C (Toluol),

7. 2-[3-[Di(2-hydroxyethyl)amino]propyl]-5-dimethylaminobenzo[de]isochinolin-1,3-dion, Fp. 180°C (Ethanol),

8. 5-Acetylamino-2-[3-[di(2-hydroxyethyl)amino]propyl]benzo[de]isochinolin-1,3-dion, Fp. 99-100°C (Wasser),

9. 5-Ethoxycarbonylamino-2-[3-[di(2-hydroxyethyl)amino]propyl]benzo[de]isochinolin-1,3-dion, Fp. 160°C (Toluol),

10. 6-(n-Butylamino)-2-[3-[di(2-hydroxyethyl)amino]propyl]benzo[de]isochinolin-1,3-dion, Fp. 152°C (Toluol),

11. 2-[2-[Di(2-hydroxyethyl)aminoethyl]-5-nitrobenzo[de]isochinolin-1,3-dion. Fp. 140°C (Methanol),

12. 2-[2-[Di(2-hydroxyethyl)amino]ethyl]-6-nitrobenzo[de]isochinolin-1,3-dion, Fp. 123°C (Ethanol),

13. 5-Amino-2-[2-[di(2-hydroxyethyl)aminoethyl]benzo[de]isochinolin-1,3-dion, Fp. 158-160°C (Methanol),

14. 6-Amino-2-[2-[di(2-hydroxyethyl)amino]ethyl]benzo[de]isochinolin-1,3-dion, Fp. 180°C (Methanol),

15. 2-[2(2-Hydroxyethyl)amino]ethyl]-6-nitro-benzo[de]isochinolin-1,3-dion, Fp. 115°C (Dimethylformamid/Wasser),

16. 5-Amino-2-[2-[(2-hydroxyethyl)amino]ethyl]benzo[de]isochinolin-1,3-dion, Fp. 160°C (Ethanol),

17. 6-Amino-2-[2-[(2-hydroxyethyl)amino]ethyl]benzo[de]isochinolin-1,3-dion, Fp. 188°C (Ethanol),

18. 2-[2-[(2-Hydroxyethyl)amino]ethyl]-5-methoxybenzo[de]isochinolin-1,3-dion, Fp. 118°C (Ethanol),

19. 2-[2-[(2-Hydroxyethyl)amino]ethyl]-6-methoxybenzo[de]isochinolin-1,3-dion, Fp. 132°C (Essigsäureethylester),

20. 6-Chlor-2-[2-[(2-hydroxyethyl)amino]ethyl]-benzo[de]isochinolin-1,3-dion, Fp. 121°C (Toluol),

21. 2-[2-[(2-Hydroxyethyl)amino]ethyl]-5-dimethylaminobenzo[de]isochinolin-1,3-dion. Fp. 241°C (Ethanol),

22. 5-Acetylamino-2-[2-[(2-hydroxyethyl)amino]ethyl]benzo[de]isochinolin-1,3-dion, Fp. 225°C (Dimethylformamid),

23. 5-Ethoxycarbonylamino-2-[2-[(2-hydroxyethyl)amino]ethyl]benzo[de]isochinolin-1,3-dion, Fp. 213°C (Dimethylformamid),

24. 6-n-Butylamino-2-[2-[(2-hydroxyethyl)amino]ethyl]benzo[de]isochinolin-1,3-dion, Fp. 178°C (Ethanol/Wasser),

## Ansprüche

1. Benzo[de]isochinolin-1,3-dione der Formel I

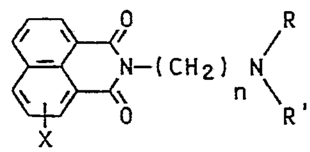

I,

worin

X eine Hydroxy-, Nitro-, $C_{1-4}$-Alkoxy-, Amino-, $C_{1-4}$-Alkylamino, Di-$C_{1-4}$-Alkylamino-, $C_{1-4}$-Alkylcarbonylamino-, $C_{1-4}$-Alkoxycarbonylamino-, $C_{1-6}$-Alkyl-oder Trifluormethylgruppe oder ein Wasserstoff-oder Halogenatom darstellt,

n die Zahl 0, 1, 2, 3 oder 4 bedeutet

R ein Wasserstoffatom oder eine Hydroxy-$C_{1-4}$-alkylgruppe ist und

R' eine Hydroxy-$C_{1-4}$-alkylgruppe darstellt,

wobei jedoch nicht X eine 5-Nitrogruppe oder ein Wasserstoffatom und n nicht 2 sein kann, wenn R ein Wasserstoffatom und R' eine Hydroxyethylgruppe darstellt, sowie deren Salze mit physiologisch verträglichen Säuren.

2. Verfahren zur Herstellung der Benzo[de]isochinolin-1,3-dione der Formel I

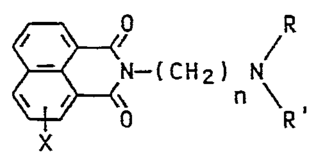

I,

worin

X eine Hydroxy-, Nitro-, $C_{1-4}$-Alkoxy-, Amino-, $C_{1-4}$-Alkylamino, Di-$C_{1-4}$-Alkylamino-, $C_{1-4}$-Alkylcarbonylamino-, $C_{1-4}$-Alkoxycarbonylamino-, $C_{1-6}$-Alkyl-oder Trifluormethylgruppe oder ein Wasserstoff-oder Halogenatom darstellt,

n die Zahl 0, 1, 2, 3 oder 4 bedeutet

R ein Wasserstoffatom oder eine Hydroxy-$C_{1-4}$-alkylgruppe ist und

R' eine Hydroxy-$C_{1-4}$-alkylgruppe darstellt,

wobei jedoch nicht X eine 5-Nitrogruppe oder ein Wasserstoffatom und n nicht 2 sein kann, wenn R ein Wasserstoffatom und R' eine Hydroxy ethylgruppe darstellt, sowie deren Salze mit physiologisch verträglichen Säuren, dadurch gekennzeichnet, daß man Naphthalsäureanhydride der Formel II

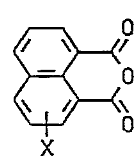

II,

worin X die angegebene Bedeutung besitzt, mit einem Amin der Formel III

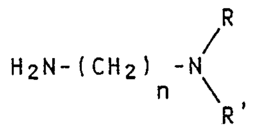

III,

worin n, R und R′ die angegebene Bedeutung besitzen, umsetzt und die so erhaltenen Verbindungen gegebenenfalls in ihre Salze mit physiologisch verträglichen Säuren überführt.

3. Verbindungen der Formel II gemäß Anspruch 1 zur Verwendung bei der Bekämpfung von Krankheiten.

4. Verwendung der Verbindungen der Formel I gemäß Anspruch 1 zur Herstellung von Arzneimitteln zur Bekämpfung von Tumoren und Leukämie.

Europäisches Patentamt

# EUROPÄISCHER RECHERCHENBERICHT

Nummer der Anmeldung

EP 87105793.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 2 423 548 (HOECHST)<br>* Anspruch 1; Beispiel 18 *<br>-- | 1 | C 07 D 221/14<br>A 61 K 33/435 |
| A | EP - A2/A3 - 0 125 439 (WARNER-LAMBERT)<br>* Ansprüche 1,4,7,8; Zusammenfassung *<br>---- | 1-4 | |

| RECHERCHIERTE SACHGEBIETE (Int. Cl 4) |
|---|
| C 07 D 221/00 |

Der vorliegende Recherchenbericht wurde für alle Patentansprüche erstellt.

| Recherchenort | Abschlußdatum der Recherche | Prüfer |
|---|---|---|
| WIEN | 31-07-1987 | HOCHHAUSER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82